# EUROPEAN PATENT SPECIFICATION

(11) **EP 1 342 473 B1**
(45) Date of publication and mention of the grant of the patent: **15.08.2007**
(21) Application number: 03012503.3
(22) Date of filing: 02.06.1997
(51) Int. Cl.: A61K 31/355, A61K 8/67, A61Q 19/08

(54) **Topical application of vitamin E esters**
Topische Anwendung von Vitamin E Ester
Application topique d' ester de vitamine E

(30) Priority: 31.05.1996 IT MI961121
(43) Date of publication of application: 10.09.2003
(62) Divisional of application: 97927104.6
(73) Proprietor: BIO.LO.GA. S.r.l., 31015 Conegliano (TV) (IT)
(72) Inventor: Panin, Giorgio, 45100 Rovigo (IT)
(74) Representative: Ferreccio, Rinaldo

(56) References cited:
- EP-A- 0 231 777
- EP-A- 0 467 218
- EP-A- 0 539 215
- EP-A- 0 579 079
- WO-A-93/03720
- US-A- 5 153 230

## Description

In general, the present invention relates to the use of esters of d,1-alpha-tocopherol, referred to below as vitamin E, with carboxylic acids and, in particular, of vitamin E acetate, in cosmetic skin treatment.

In particular, the present invention relates to the topical application of the aforesaid esters to the skin for increasing the moisture content of human skin.

Vitamin E and its derivatives are substances which, by virtue of their presumed properties as antioxidants and removers of free radicals, are widely used in the cosmetics industry in the preparation of formulations for combatting or preventing unsightly skin conditions.

For these reasons, vitamin E and its derivatives are often present in cosmetic formulations, in concentrations variable from 0.5 to 10%, as "active" components with antioxidant and anti-ageing effects.

In fact, the antioxidant and anti-ageing effect of vitamin E at skin level has never been scientifically demonstrated.

Compositions for topical use containing up to 20% by weight (preferably up to 10%) of vitamin E in combination with other active ingredients, emulsifiers and excipients are described in application EP-A-0 158 090. In this application, it is maintained that the aforesaid compositions are useful in the treatment of eczema, skin inflammation and irritation, skin allergies, skin pigmentation, etc., although experimental support is supplied solely for the application of ointments containing 8% of non-esterified vitamin E for the prevention of erythema due to exposure to the sun or to radiation.

WO 93/03720 discloses a method of treating sunburns in human or animal skin, comprising the application to the sunburned area of a topical composition comprising as active ingredient a tocopherol ester, e.g. tocopherol acetate.

EP-A-0 467 218 discloses a lipid combination for cosmetic purposes, containing up to 95% of unsaturated fatty acids of tocopheryl esters thereof together with at least one further component selected from n-alkanes, squalene, cholesterol, triglycerides and-wax esters.

EP-A-0 231 777 describes a cosmetic agent for topical application, including an effective amount of a moisturizing substance and comsetically acceptable carrier substances, wherein the moisturizing substance is a mixture of a linoleic acid ester of alpha-tocopherol and other fatty acid esters of alpha-tocopherol.

EP-A-O 579 079 discloses a cosmetic agent containing vitamin E or derivatives thereof together with 2-(dihydroxyethyl)2-hydroxy-6,10,1-4-trimethyl-pentadecane, conventional carriers and adjuvants and optionally a UV-filter substance.

EP-A-O 539 215 describes a method of enhancing the penetration- of a topically applied composition comprising a therapeutically active agent which comprises including in the composition a penetration-enhancing amount of vitamin E.

US-A- 5 153 230 discloses a topical skin cream composition comprising an organic acid, such as e.g. glycolic acid, vitamin A palmitate, up to 5% vitamin E acetate and a diluent or carrier.

The present invention was arrived at in the first place by investigation of the cosmetic properties of the esters of vitamin E when applied topically in the absence of other active ingredients or excipients, particularly those of vitamin E acetate, which is a clear, yellow, viscous liquid, which is insoluble in water, resistant to oxidation by air, by light and by UV rays, and thermally stable.

The human skin behaves as a double barrier, preventing the entry of substances from the outside atmosphere and regulating the passage of water and electrolytes towards the exterior. This barrier function is performed mainly by the cornified layer of the epidermis by means of the corneocyte component which is rich in fibrous proteins (keratin, filagrin, loricrin) and the intercorneocyte lipid component (cholesterol, free fatty acids and ceramides) synthesized by the cells of the granular layer.

At the level of the cornified layer, the ceramides, together with the cholesterol and its esters and the free fatty acids, fill the intercellular space and form a continuous fatty film, defining a barrier to the entry of foreign hydrophilic substances.

Damage to the epidermal barrier function opens the way through the epidermis for electrolytes and chemical substances.

This damage may occur either as a result of the use of toxic or aggressive substances or, more simply, because of too frequent washing due to incorrect habits or to frequent sports or recreational activities.

The consequences of this skin damage may consist of irritative dermatitis or even skin infections.

There is therefore a need to provide a barrier product which offers the skin good protection from attack by irritants and is well-tolerated and easily applied, given that it may be used repeatedly during the day.

An effective barrier product is important in occupational medicine for preventing work-related dermatitis, in the dermatological field for preventing conditions caused by damage to the barrier function (particularly due to irritative or allergy causes), and in the paediatric field, in which skin protection is required basically because the structure of the barrier function is incomplete and the skin is more easily damaged.

To satisfy the aforementioned requirement, the prior art has provided formulations for topical use which can form a kind of barrier on the skin against attack by external agents and can keep the skin moisturized.

These topical formulations are essentially of two basic types: hydrophobic ointments and barrier creams.

A hydrophobic ointment is a single-phase system normally constituted by a semi-solid matrix (Vaseline, paraffin, silicones with high molecular weights), by a liquid consistency regulator (Vaseline oil, vegetable oils, liquid silicones, synthetic esters) and by a stabilizer (beeswax or its synthetic substitutes).

These ointments are used because of their properties of blocking or inhibiting the normal evaporation of water from the skin, causing increased skin moisturization.

However, the aforesaid ointments have the serious disadvantage of leaving the skin greasy and of feeling to the user like a foreign substance generating a need for cleansing.

Moreover, the mineral oils contained in hydrophobic ointments are hydrocarbons derived from petroleum and, although subjected to purification processes, still contain contaminants, particularly polycyclic aromatic hydrocarbons, which are considered responsible for some contact dermatitis and have carcinogenic effects.

Finally, hydrophobic ointments have poor thermal stability.

A barrier cream is an emulsion comprising a lipophilic phase, generally constituted by 20-30% of Vaseline or paraffin and 60-70% of an aqueous phase, with the addition of stabilizers (surfactants, preservatives) and moisturizers.

These creams are more pleasant to apply than hydrophobic ointments because they are characterized by less greasiness, but are also less effective.

Their barrier effect and their moisturizing effect are in fact due essentially to the lipophilic phase they contain, since the aqueous phase evaporates from the skin almost immediately and the moisturizing components they contain do not have proven effectiveness.

The latter components may be hygroscopic substances such as glycerine, sorbitol, polyethylene glycols and polypropylene glycols, but the effectiveness of these substances is doubtful since they do not modify the water-retention capacity of the cornified layer because they are molecules foreign to the protein and lipid molecules of this layer which have the physiological function of binding water.

Other moisturizing components are constituted by substances which interact chemically with the keratin of the cornified layer, such as urea and the alpha-hydroxy acids; however, these substances, particularly if used in high concentrations, may show keratolytic effects, possibly inducing erythema and/or subjective boring pain sensations.

Finally, substances such as collagen, the glycosaminoglycanes and elastin have been proposed as moisturizing agents and effectively perform a water-retention function in the human skin but, on the skin, can at most perform a humectant function. Some of these substances, particularly if they are of animal origin, like collagen, may contain contaminants and are potentially allergenic.

In conclusion, there is currently no formulation with a barrier and moisturizing effect which is simultaneously effective, pleasant to apply and safe from the toxicological and allergy points of view.

The problem on which the present invention is based is that of providing a new preparation for topical application with a barrier effect and with moisturizing activity which does not have the disadvantages shown by the preparations of the prior art.

This problem is solved, according to the invention, by a method for increasing the moisture content of human skin, consisting of the topical application, of an ester of vitamin E with a carboxylic acid of formula R-COOH, in which R is an alkyl, alkenyl or alkynyl radical having from 1 to 19 carbon atoms, free of any other substance.

The ester is preferably vitamin E acetate.

The term vitamin E is intended to mean both alpha-tocopherol in racemic form (d, 1) and the individual enantiomers and mixtures thereof.

It has been found experimentally that vitamin E acetate applied to the skin as such without the addition of any stabilizers, consistency regulators, preservatives, etc., (this product will be referred to below as "pure vitamin E acetate") achieves a barrier effect and a moisturizing effect equal to if not better than those achieved by hydrophobic ointments, without having the disadvantages of the latter.

Pure vitamin E acetate is in fact particularly easy to spread, is absorbed surprisingly quickly, produces no unpleasant thermal sensations, leaves the skin shiny only for a few minutes and then leaves it soft, elastic and not sticky, and resists cleansing with water or detergents.

By virtue of these properties, it is also possible to increase the moisture content of human skin by using pure vitamin E acetate as an after-sun product and as a topical product after recreational or sports activities which may compromise the integrity of the cornified layer of the epidermis.

Moreover, since vitamin E acetate is not a molecule foreign to the human organism, it can easily integrate with the lipids present in the cornified layer.

To evaluate the absorption of pure vitamin E acetate, subjective tests were carried out in 20 individuals aged from 25 to 60 years.

The pure vitamin E acetate was pleasant to apply owing to absence of odour, a soft feel of the skin, absence of greasiness, absence of a feeling that a foreign substance had been applied and consequently absence of a need for cleansing, lack of side effects during absorption (no burning of the skin, no boring pain sensation, even with irritated skin).

The application of pure vitamin E acetate, which is very easy per se, can be further facilitated and rendered as uniform as possible by spray dispensers.

In this case, the vitamin E acetate is dissolved in volatile diluents which evaporate instantaneously or within a few seconds after the application of the spray, leaving a film solely of vitamin E acetate on the skin.

Preferably, spray dispensing systems which do not use organic volatile or gaseous compounds as propellants are used, such as, for example, the EP SPRAY SYSTEM® from the Swiss firm EP SPRAY SYSTEM S.A., which uses compressed air.

Two minutes after the application of the pure vitamin E acetate, the skin had no residual greasiness.

The moisturizing effect of the pure vitamin E acetate was evaluated experimentally by corneometry.

Pure vitamin E acetate was applied topically twice a day to 10 healthy subjects aged between 25 and 45 years in two different regions on the skin, of which one was exposed (the back of the left hand) and one was not exposed (the right forearm).

Four measurements were made in each region every hour for 12 hours, for five consecutive days.

From the data obtained it was possible to establish a clear improvement in skin moisturization and, in particular, persistence of the moisturization for at least 12 hours.

Tolerance to pure vitamin E acetate was also tested by patch tests carried out on 20 healthy subjects aged between 25 and 45 years, of whom 2 had positive anamnesis owing to atopy.

None of the subjects examined showed positive reaction to the patch tests.

A comparative test was then carried out to evaluate the moisturizing effect of pure vitamin E acetate in comparison with Vaseline oil and with a commercial cream base, that is, the product IDIBASE®.

The moisturizing effect was evaluated by skin corneometry. 5 female subjects were tested and were asked to apply the vitamin E acetate to the back of the left hand and to the central surface of the left forearm every 12 hours with slight massage.

Vaseline oil was applied in the corresponding regions on the right-hand side and in the same manner. The cream base IDIBASE® was applied to one forearm in a different region.

The subjects were tested for a period of three days; they washed their hands frequently with aggressive soaps, according to their occupation, whereas the forearm was washed only every 24 hours.

The determinations were carried out by means of corneometers, that is, by a special capacitance meter which measures the variations in the capacitance of the skin induced by a greater or lesser water content of the more superficial layers of the epidermis, by means of a skin probe.

Each day a basal measurement (Tₒ) was made followed by measurements 3 minutes, 4 hours and 10 hours after application.

The average of the measurements gave the results summarized in Table 1 below.

**TABLE 1**

| (average of corneometric values) | | | | | | | | | |
|---|---|---|---|---|---|---|---|---|---|
| Vitamin E acetate | | | | Vaseline oil | | | | IDIBASE® | |
| left hand | | left forearm | | right hand | | right forearm | | Left forearm | |
| Tₒ | 55 | Tₒ | 55 | Tₒ | 56 | To | 55 | Tₒ | 66 |
| 3m | 49 | 3m | 39 | 3m | 47 | 3m | 58 | 3m | 69 |
| 5h | 59 | 5h | 58 | 5h | 57 | 5h | 40 | 5h | 56 |
| 10h | 61 | 10h | 60 | 10h | 56 | 10h | 57 | 10h | 65 |

It can be inferred from an analysis of the values given in Table 1 that pure vitamin E acetate brought about a considerable increase in skin moisturization, whereas the products used for comparison did not modify it substantially.

Essentially similar results were obtained by the topical application of vitamin E linoleate.

Finally, the persistence of the pure vitamin E acetate on the skin was measured in order to have an indication of its protective effect.

The same subjects who were subjected to corneometry were also subjected to a sebometric test carried out by means of a sebometer, that is, a device which can determine by a photometric method how much cutaneous fat is deposited on a semitransparent tape with which the device is provided.

The tape is applied to the preselected region for a standard time (30 seconds) with a constant pressure.

The more cutaneous fat is deposited on the tape the more transparent it becomes and the higher will be the values detected by the sebometer.

This type of test is normally used to evaluate the amount of sebum present on the skin; in the case of the present invention it was used to evaluate the persistence of the oily substance applied to the skin, even after the skin had been washed many times.

The results obtained are summarised in Table 2.

**TABLE 2**

| (average of the sebometric values) | | | | | | | | | |
|---|---|---|---|---|---|---|---|---|---|
| Vitamin E acetate | | | | Vaseline oil | | | | IDIBASE® | |
| left hand | | left forearm | | right hand | | right forearm | | Left forearm | |
| To | 10 | To | 6 | To | 10 | To | 13 | To | 5 |
| 3m | 340 | 3m | 330 | 3m | 308 | 3m | 304 | 3m | 220 |
| 5h | 30 | 5h | 60 | 5h | 50 | 5h | 29 | 5h | 7 |
| 10h | 20 | 10h | 44 | 10h | 22 | 10h | 10 | 10h | 2 |

The data in Table 2 show a prolonged persistence of the pure vitamin E acetate on the skin even after repeated washing of the hands. It is perceived by the sebometer as a high presence of skin "greasiness" but this greasiness is not in fact perceived by the subject as a troublesome condition, although it represents a valuable protective film.

Substantially similar results were achieved by the topical application of vitamin E linoleate.

## Claims

1. A non-therapeutic method for increasing the moisture content of human skin consisting of the topical application of an ester of vitamin E with a carboxylic acid of formula R-COOH, in which R is an alkyl, alkenyl or alkynyl radical having from 1 to 19 carbon atoms, free of any other substance.

2. A method according to claim 1, wherein said ester of vitamin E is vitamin E acetate.

3. A method according to any of claims 1- and 2, **characterized in that** the application is carried out at least twice a day.

4. A method according to any of claims 1 to 3, wherein the topical application is carried out by spray dispensers.

## Patentansprüche

1. Nicht-therapeutisches Verfahren zur Erhöhung des Feuchtigkeitsgehalts von menschlicher Haut bestehend aus der lokalen Anwendung eines Vitamin E Esters mit einer Carbonsäure der Formel R-COOH, wobei R ein Alkyl, Alkenyl oder Alkynyl Radikal ist, das 1 bis 19 Kohlenstoffatome hat, frei von jeder anderen Substanz.

2. Verfahren nach Anspruch 1, wobei der Vitamin E Ester ein Vitamin E Acetat ist.

3. Verfahren nach einem der Ansprüche 1 und 2, **dadurch gekennzeichnet, dass** die Anwendung zumindest zweimal täglich ausgeführt wird.

4. Verfahren nach einem der Ansprüche 1 bis 3, wobei die lokale Anwendung mit einem Sprühdispenser erfolgt.

## Revendications

1. Un procédé non thérapeutique pour augmenter le taux d'hydratation de la peau humaine, constitué par l'application topique d'un ester de vitamine E avec un acide carboxylique de formule R-COOH, où R est un radical alkyle, alcényle ou alcynyle ayant de 1 à 19 atomes de carbone, en l'absence de toute autre substance.

2. Un procédé selon la revendication 1, où ledit ester de vitamine E est un acétate de vitamine E.

3. Un procédé selon l'une des revendications 1 et 2, **caractérisé en ce que** l'application est effectuée au moins deux fois par jour.

4. Un procédé selon l'une des revendications 1 à 3, **caractérisé en ce que** l'application topique est effectuée par des distributeurs à pulvérisation.
